# EUROPEAN PATENT APPLICATION

(11) **EP 4 847 080 A1**
(43) Date of publication of application: **02.12.2026**
(21) Application number: 24834220.6
(22) Date of filing: 24.01.2024
(51) Int. Cl.: A61N 1/36

(54) **PHYSIOTHERAPY DEVICE FOR TENOSYNOVITIS**

(71) Applicant: Guangzhou New Design Biotechnology Co., Ltd, Guangzhou, Guangdong 510220 (CN)
(72) Inventor: ZHANG, Shaowei, Guangzhou, Guangdong 510220 (CN); ZOU, Sile, Guangzhou, Guangdong 510220 (CN); SHEN, Weijie, Guangzhou, Guangdong 510220 (CN)
(74) Representative: Zaboliene, Reda
(86) International application number: PCT/CN2024/073845
(87) International publication number: WO 2025/156155

(57) **Abstract**

A physiotherapy instrument for tenosynovitis, including a wristband assembly, a finger kit, an electric control assembly, an electrical stimulation assembly. The wristband assembly includes a wrist strap. The finger kit includes a finger stall strap, the finger stall strap is connected to the wrist strap and encloses a finger part where a thumb passes through. The electric control assembly includes a magnetic metal terminal and a controller, the magnetic metal terminal is mounted on the wrist strap. The controller is magnetically connected and electrically communicated with the magnetic metal terminal. The electrical stimulation assembly includes two conductive bands, the two conductive bands are mounted on the surface of the wrist strap, one end of the two conductive bands is electrically connected to the magnetic metal terminal, the other end of the two conductive bands extends into the finger part to fit the inner surface of the finger part.

## Description

### CROSS-REFERENCE TO RELATED APPLICATIONS

This application is a continuation of International Application No. PCT/CN2024/073845, filed on January 24, 2024. All of the aforementioned applications are incorporated herein by reference in their entireties.

### TECHNICAL FIELD

This application relates to the technical field of medical physiotherapeutic instruments, in particular to a physiotherapy instrument for tenosynovitis.

### BACKGROUND

Tendon sheath is a sheath-like structure wrapping a tendon, with the outer layer being fiber tissues attached to bones and adjacent tissues to play roles of fixing and protecting the tendon. The inner layer is a synovial membrane that can nourish the tenon and secrete synovial, which contributes to the sliding of the tenon. However, due to long-term repeated excessive injury, the tenon and tendon sheath will be subjected to inflammation, edema, and thickening of a fiber sheath wall to form a narrow ring. Fibrosis and thickness of the tenon lead to sliding difficulty of the tenon in the sheathing canal, thereby resulting in stenosing tenosynovitis. A patient with stenosing tenosynovitis will feel joint pain. Affected joints will swell and even snap, and have the movement disorder. The joint movement disorder will be induced easily without treatment for a long time, which severely affects life and work of the patient.

Physiotherapies for tendon sheath of wrists are usually heating treatment. For example, a Chinese patent No. CN202211318092.9 discloses an acupuncture point massage device for thumb tenosynovitis. By arranging a heating sheet side pocket at a lower middle edge of the inner side surface of a main body part of a wrist strap, a heating patch or a steam patch is placed and replaced in the device according to a treatment need to alleviate contracture and pain of the wrist. However, the device is common in heating curative effect.

A pair of gloves for treating tenosynovitis is disclosed in current disclosure such as CN201822183207.3, which uses a method of electrical stimulation for physiotherapy of the wrist, or a wrist kit device for acupuncture and moxibustion therapy of tenosynovitis disclosed by CN219184932U uses a method for acupuncture-therapy of the wrist. However, all the devices need structures such as a fixed supporting part, are fixed in shape, and are inconveniently folded and carried. Moreover, the physiotherapeutic mode performs electrode or acupuncture counterpoint treatment on each acupuncture point of the wrist, which has the problem of misaligning the acupuncture points or not fully covering the acupuncture points of the finger and wrist, with local physiotherapy.

### SUMMARY

This application provides a physiotherapy instrument for tenosynovitis, which is convenient to carry and can perform electrical muscle stimulation physiotherapy on acupuncture points of the thumb and wrist comprehensively, so as to improve the physiotherapeutic effect.

The technical solution used in this application is as follows: a physiotherapy instrument for tenosynovitis, including a wristband assembly, a finger kit, an electric control assembly, and an electrical stimulation assembly. The wristband assembly includes a wrist strap. The finger kit includes a finger stall strap, and the finger stall strap is connected to the wrist strap and encloses a finger part where a thumb passes through. The electric control assembly includes a magnetic metal terminal and a controller, and the magnetic metal terminal is mounted on the wrist strap. The controller is magnetically connected and electrically communicated with the magnetic metal terminal. The electrical stimulation assembly includes two conductive bands, the two conductive bands are mounted on the surface of the wrist strap, one end of each of the two conductive bands is electrically connected to the magnetic metal terminal, and the other end of each of the two conductive bands extends into the finger part to fit the inner surface of the finger part. During physiotherapy, the finger part is sleeved on the thumb, the wrist strap wraps around the periphery of the wrist, the conductive bands are attached to peripheral surfaces of the wrist and the thumb to electrically stimulate the peripheral surfaces of the wrist and the thumb.

Further, the wrist strap includes a first strap layer and a second strap layer, the magnetic metal terminal is clamped between the first strap layer and the second strap layer, the magnetic metal terminal is provided with a connecting protrusion exposed outside of the first strap layer, a connecting terminal is arranged at the bottom of the controller, and the connecting terminal is in magnetic contact with the connecting protrusion of the magnetic metal terminal.

Further, the electric stimulation assembly includes a conductive fixed part, and one end of each of the conductive bands is mounted on the wrist strap through the conductive fixed part.

Further, the electric stimulation assembly includes a wire, the wire is clamped between the first strap layer and the second strap layer, and the wire conducts the conductive fixed part and the magnetic metal terminal.

Further, the physiotherapy instrument for tenosynovitis includes a heating assembly, where the heating assembly includes a heating sheet, the heating sheet is clamped between the first strap layer and the second strap layer, and the magnetic metal terminal is electrically connected to the heating sheet.

Further, the wristband assembly includes a hair side magic tape and a hook side magic tape, the hair side magic tape is arranged on the surface of the first strap layer and the hook side magic tape is arranged on the second strap layer, so that when the wrist strap wraps around the wrist, the hook side magic tape and the hair side magic tape are adhered.

Further, the first strap layer and the second strap layer both are cloth layers.

Further, both end parts of the finger part respectively extend at a preset length and are respectively attached to the surface of the wrist strap, one end of each of the two conductive bands is attached to the extended section corresponding to each end part of the finger part, and the other end extends into the finger part to fit the inner surface of the finger part.

Further, the electrical stimulation assembly includes a metal rivet, one end of each of the conductive bands and one end of the finger stall strap away from the finger part are riveted to the wrist strap through the metal rivet, and the other end of each of the conductive bands and one end of the finger stall strap close to the finger part are integrally spliced to the surface of the wrist strap.

Further, the controller is provided with a switch button, an electrical stimulation gear adjusting button, and a heating gear adjusting button.

Compared with the prior art, according to the physiotherapy instrument for tenosynovitis provided by this application, by arranging the two conductive bands mounted on the surface of the wrist strap wrapping around the wrist, one end of each of the conductive bands extends into the finger part to fit the inner surface of the finger part at one end of the wrist strap. During physiotherapy, the finger part is sleeved on the thumb, the wrist strap wraps around the periphery of the wrist, and the two conductive bands can be attached to the peripheral surfaces of the wrist and thumb to electrically stimulate the peripheral surfaces of the wrist and thumb at the same time. By arranging the strip-like conductive bands on the wrist strap and extending the conductive bands in the finger stall, the acupuncture points of the thumb and wrist can be comprehensively coated, so that the acupuncture points of the thumb and wrist can be comprehensively subjected to electric muscle stimulation physiotherapy at the same time. Therefore, the physiotherapeutic effect is effectively improved.

### BRIEF DESCRIPTION OF THE DRAWINGS

Drawings are used to provide a further understanding of this application and constitute a part of the specification. Together with the specific implementations below, the drawings are used for explaining this application, rather than limiting this application. In the drawings:
Fig. 1 is a three-dimensional constitutional diagram of a physiotherapy instrument for tenosynovitis provided by this application;
Fig. 2 is another three-dimensional constitutional diagram of a physiotherapy instrument for tenosynovitis provided by this application;
Fig. 3 is a three-dimensional exploded view of a physiotherapy instrument for tenosynovitis provided by this application;
Fig. 4 is another three-dimensional exploded view of a physiotherapy instrument for tenosynovitis provided by this application; and
Fig. 5 is a state diagram of a physiotherapy instrument for tenosynovitis provided by this application during use.

### Numerals in the drawings:

1, controller; 101, connecting terminal; 102, switch button; 103, electrical stimulation gear adjusting button; 104, heating gear adjusting button; 2, heating sheet; 3, magnetic metal terminal; 301, protrusion; 4, first strap layer; 5, second strap layer; 6, wire; 7, hair side magic tape; 8, hook side magic tape; 9, finger stall strap; 901, finger part; 10, conductive band; 11, conductive fixed part.

### DETAILED DESCRIPTION OF THE EMBODIMENTS

The specific implementation of this application is described in detail below in conjunction with the drawings. It should be understood that the specific implementations described herein are merely used to describe and explain this application, but are not intended to limit this application.

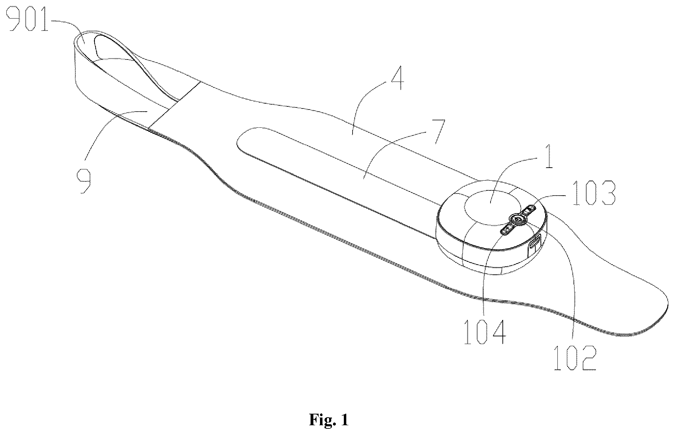

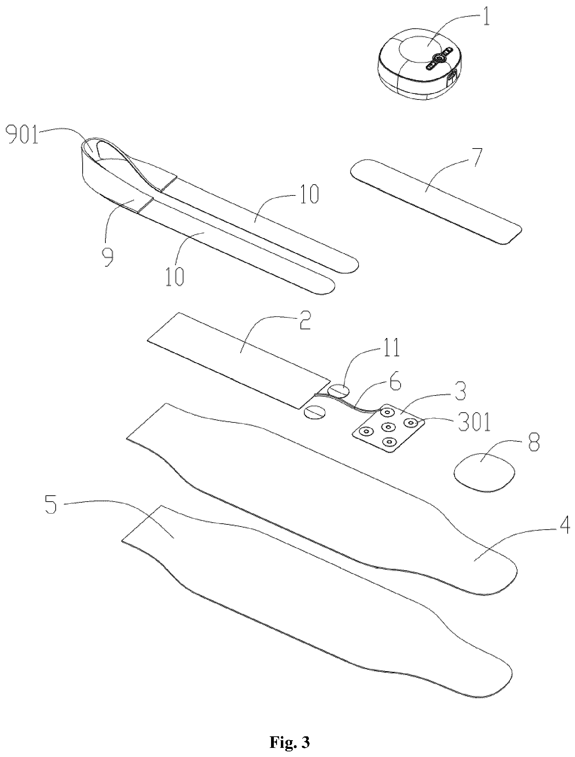

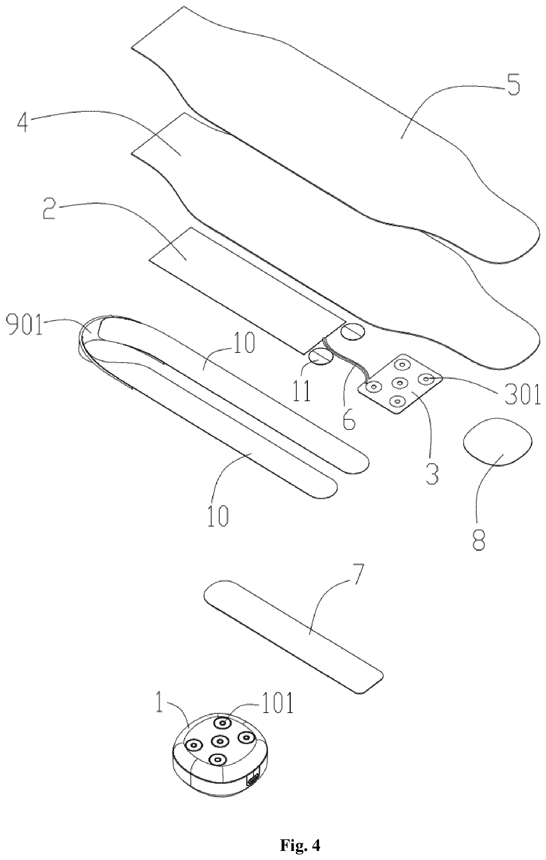

As shown in FIGS. 1-4, a physiotherapy instrument for tenosynovitis provided by this application includes a wristband assembly, a finger kit, an electric control assembly, an electrical stimulation assembly, and a heating assembly.

The wristband assembly includes a wrist strap, where the wrist strap includes a first strap layer 4 and a second strap layer 5.

The finger kit includes a finger stall strap 9, and the finger stall strap 9 is connected to the wrist strap and encloses a finger part 901 where a thumb passes through.

The electric control assembly includes a magnetic metal terminal 3 and a controller 1, and the magnetic metal terminal 3 is mounted on the wrist strap, to be electrically connected to the electrical stimulation assembly and the heating assembly. The controller 1 is mounted on the magnetic metal terminal 3 to control the electrical stimulation assembly and the heating assembly.

The electrical stimulation assembly includes two conductive bands 10: a positive electrode conductive band and a negative electrode conductive band. The two conductive bands 10 are mounted on the surface of the wrist strap, one end of each of the two conductive bands 10 is electrically connected to the magnetic metal terminal 3, the other end of each of the two conductive bands extends into the finger part 901 to fit the inner surface of the finger part 901, and the end parts of the two conductive bands 10 extending into the finger part 901 are not connected. During physiotherapy, the finger part 901 is sleeved on the thumb, the wrist strap wraps around the periphery of the wrist, the conductive bands 10 are attached to peripheral surfaces of the wrist and the thumb to electrically stimulate the peripheral surfaces of the wrist and the thumb.

The heating assembly includes a heating sheet 2.

In the embodiment, the magnetic metal terminal 3 and the heating sheet 2 both are clamped between the first strap layer 4 and the second strap layer 5, and the magnetic metal terminal 3 is electrically connected to the heating sheet 2. The controller 1 is removably mounted on the outer surface of the first strap layer 4. Specifically, a plurality of connecting protrusions 301 are arranged on the surface of the magnetic metal terminal 3, the connecting protrusions 301 are exposed outside the first strap layer 4, the connecting terminal 101 is correspondingly arranged at the bottom of the controller 1, the connecting terminal 101 can be an inserting groove structure matching with the connecting protrusions 301, the connecting terminal 101 is in magnetic contact with the connecting protrusions 301 of the magnetic metal terminal 3 and is electrically communicated with the same, so that the controller 1 and the magnetic metal terminal 3 are removably connected. The controller 1 controls the electrical stimulation assembly and the heating assembly, so that the conductive bands 10 perform electrical stimulation on the peripheral surfaces of the wrist and thumb. The heating sheet 2 is used for hot compressing and thermal therapy of the wrist and thumb of the patient. In addition, the controller 1 is provided with a switch button 102, an electrical stimulation gear adjusting button 103, and a heating gear adjusting button 104.

When heating physiotherapy or electrical stimulation physiotherapy is needed, the controller 1 is magnetically fixed with the connecting protrusions of the magnetic metal terminal 3. The controller 1 is opened by pressing the switch button 102. Then the electrical stimulation gear adjusting button 103 or the heating gear adjusting button 104 is pressed to perform physiotherapy with corresponding functions. By continuously pressing the electrical stimulation gear adjusting button 103 or the heating gear adjusting button 104 at number of times to adjust the gear, for example, the button is pressed once for a first gear (weak electrical stimulation/mild heat), the button is pressed twice for a second gear (strong electrical stimulation/moderate heat), by parity of reasoning.

When physiotherapy is not needed, the switch button 102 is only pressed to close the controller 1 or remove the controller 1 directly from the first strap layer 4 to power off. Moreover, after the controller 1 is removed from the wrist strap, the wrist strap still has the wrist protecting effect.

The first strap layer 4 and the second strap layer 5 both are cloth layers, leather layers or woven fabrics. The magnetic metal terminal 3 is connected to the heating sheet 2 through the wire 6 or the magnetic metal terminal 3 and the heating sheet 2 are arranged integrally and are in direct contact.

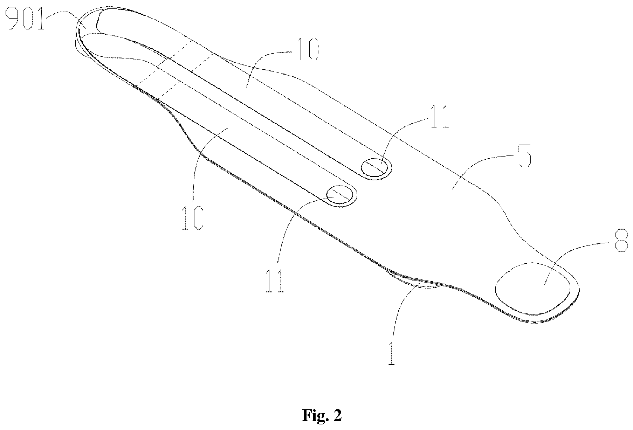

Referring to Fig. 1 and Fig. 2 again, further, the wristband assembly includes a hair side magic tape 7 and a hook side magic tape 8. The hair side magic tape 7 is arranged on the surface of the first strap layer 4, and the hook side magic tape 8 is arranged on the second strap layer 5. After the wrist strap winds the wrist of the patient, the hair side magic tape 7 and the hook side magic tape 8 are attached to each other, so that the wrist strap is worn at the wrist of the patient. It could be understood that in other embodiments, the wrist strap can also be worn at the wrist of the patient by arranging structures such as a magnetic buckle and a fastener on the first strap layer 4 and the second strap layer 5, which is not limited herein.

As shown in FIGS. 2-4, in the embodiment, the finger stall strap 9 is a short strip, both ends of which are connected to the wrist strap and enclose the finger part 901 where the thumb passes through. The finger part 901 is formed at one end of the wrist strap to be sleeved on the thumb. The two conductive bands 10 are mounted on the surface of the second strap layer 5, and at least part of each of the conductive bands 10 extends to the finger part 901. Further, the electrical stimulation assembly includes a conductive fixed part 11. The two conductive bands 10 are fixed to the wrist strap through the conductive fixed part 11, and the conductive fixed part 11 is electrically communicated with the magnetic metal terminal 3. Therefore, the two conductive bands 10 and the magnetic metal terminal 3 are electrically connected. Specifically, the conductive fixed part 11 can be electrically connected to the magnetic metal terminal 3 directly or can be electrically communicated and connected to the magnetic metal terminal through the wire 6. In the embodiment, one end of each of the conductive bands 10 is fixed to the second strap layer 5 through the conductive fixed part 11 and the other end, i.e., the end close to the finger part 901, is spliced to the second strap layer 5 through a sewing thread. Preferably, the conductive fixed part 11 is a metal rivet.

In another preferred implementation, the finger stall strap 9 is a long strip, i.e., both end parts of the finger part 901 respectively extend at a preset length and are respectively attached to the surface of the wrist strap, where the preset length can be a length winding at least one circle of the wrist. One end of each of the two conductive bands 10 is attached to the extended section corresponding to each end part of the finger part 901, and the other end extends into the finger part 901 to fit the inner surface of the finger part 901. The conductive fixed part 11 is a metal rivet. One end of each of the conductive bands 10 and one end of the finger stall strap 9 away from the finger part 901 are riveted to the wrist strap through the metal rivet, and the other end of each of the conductive bands 10 and one end of the finger stall strap 9 close to the finger part 901 are integrally spliced to the surface of the wrist strap.

Further, in order to prevent the top end surface of the metal rivet from being exposed outside the surface of the second strap layer 5, the top end surface of the metal rivet is sheltered by sealing a seal cover.

The finger stall strap 9 is made of a material with better extensibility, which can bear a large pull such as a Leica reel and a resistance band. The conductive bands 10 are made of a conductive silica gel or conductive graphene; and the conductive fixed part 11 is any article capable of being conductive and fixed such as the metal rivet, a metal screw or a conductive adhesive tape, which are not limited herein.

Further, at least part of the conductive fixed part 11 passes through the second strap layer 5 and is located between the first strap layer 4 and the second strap layer 5. A conductive element (not shown in the drawings) is arranged between the first strap layer 4 and the second strap layer 5, and the conductive element is connected to the conductive fixed part 11 and the magnetic metal terminal 3 to connect the controller 1 and the two conductive bands 10. The conductive element is the wire 6, a conductive fabric or another conductive apparatus, which is not limited herein. The controller 1 controls the two conductive bands 10 to perform electrical muscle stimulation (EMS) on the thumb of the patient, so that the physiotherapeutic effect of the physiotherapy instrument is effectively improved.

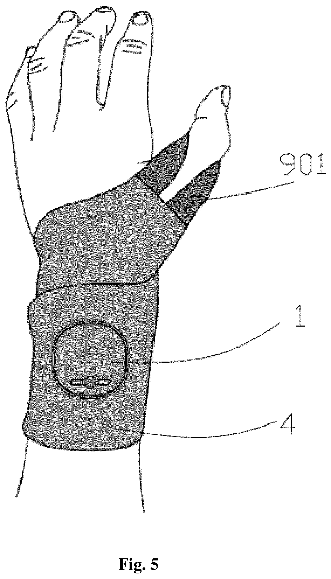

Referring to Fig. 5, when the physiotherapy instrument for tenosynovitis provided by this application is worn, the thumb passes through the finger part 901 first, then the wrist strap winds the wrist of the patient, and finally, the hair side magic tape 7 and the hook side magic tape 8 are attached to each other, so that the wrist strap is worn at the wrist of the patient; in this case, the two conductive bands 10 directly cover the acupuncture points around the wrist and thumb of the patient, for example, ashi acupuncture point, shenmen point, wangu point, yanggu point, yangchi point, yuji point and the like, to perform electrical stimulation physiotherapy on these acupuncture points; when hot compressing is needed, the controller 1 starts heating, the heating sheet 2 heats, and the physiotherapy instrument for tenosynovitis can execute independent hot compressing and thermal therapy or independent electrical stimulation physiotherapy and can perform hot compressing and thermal therapy and electrical stimulation physiotherapy at the same time.

In conclusion, the physiotherapy instrument for tenosynovitis provided by this application has the following advantages:
By arranging the finger part 901 at one end of the wrist strap, the conductive bands 10 are attached to the surface of the wrist strap and extend to the finger part 901 to fully cover the finger and wrist comprehensively, so as to perform electrical muscle stimulation physiotherapy on the wrist and thumb at the same time, so that the physiotherapeutic effect is effectively improved.

By arranging the heating sheet 2, the heating sheet 2 is connected to the magnetic metal terminal 3, so that the physiotherapy instrument has not only the electrical stimulation physiotherapeutic function, but also hot compressing and thermal therapy functions, so that the physiotherapeutic effect is further improved.

By arranging the removable controller 1, when physiotherapy is needed, the controller 1 is mounted at the magnetic metal terminal 3; and when physiotherapy is not needed, the controller 1 is removed, and the wrist strap and the finger part 901 still have the function of protecting the wrist and finger, without affecting independent use of the wrist strap. Moreover, when the wrist strap is not used, it is convenient to fold and store, and the wrist strap and the controller 1 can be carried respectively and are convenient to carry.

Various different embodiments of this application are arbitrarily combined without departing from the thought of the creation of this application, which all should be regarded as the content disclosure in this application. Within the technical concept range of this application, various simple variations on the technical solution and arbitrary combinations on the different embodiments without departing from the thought of the creation of this application should fall within the protection scope of this application.

## Claims

1. A physiotherapy instrument for tenosynovitis, comprising a wristband assembly, a finger kit, an electric control assembly, and an electrical stimulation assembly, wherein
the wristband assembly comprises a wrist strap;
the finger kit comprises a finger stall strap (9), and the finger stall strap (9) is connected to the wrist strap and encloses a finger part (901) where a thumb passes through;
the electric control assembly comprises a magnetic metal terminal (3) and a controller (1), the magnetic metal terminal (3) is mounted on the wrist strap, and the controller (1) is magnetically connected and electrically communicated with the magnetic metal terminal (3);
the electrical stimulation assembly comprises two conductive bands (10), the two conductive bands (10) are mounted on the surface of the wrist strap, one end of each of the two conductive bands (10) is electrically connected to the magnetic metal terminal (3), and the other end of each of the two conductive bands extends into the finger part (901) to fit the inner surface of the finger part (901); and
during physiotherapy, the finger part (901) is sleeved on the thumb, the wrist strap winds the periphery of the wrist, and the conductive bands (10) are attached to peripheral surfaces of the wrist and the thumb to electrically stimulate the peripheral surfaces of the wrist and the thumb.

2. The physiotherapy instrument for tenosynovitis according to claim 1, wherein the wrist strap comprises a first strap layer (4) and a second strap layer (5), the magnetic metal terminal (3) is clamped between the first strap layer (4) and the second strap layer (5), the magnetic metal terminal (3) is provided with a connecting protrusion (301) exposed outside of the first strap layer (4), a connecting terminal (101) is arranged at the bottom of the controller (1), and the connecting terminal (101) is in magnetic contact with the connecting protrusion (301) of the magnetic metal terminal (3).

3. The physiotherapy instrument for tenosynovitis according to claim 2, wherein the electric stimulation assembly comprises a conductive fixed part (11), and one end of each of the conductive bands (10) is mounted on the wrist strap through the conductive fixed part (11).

4. The physiotherapy instrument for tenosynovitis according to claim 3, wherein the electric stimulation assembly comprises a wire (6), the wire (6) is clamped between the first strap layer (4) and the second strap layer (5), and the wire (6) conducts the conductive fixed part (11) and the magnetic metal terminal (3).

5. The physiotherapy instrument for tenosynovitis according to claim 1, comprising a heating assembly, wherein the heating assembly comprises a heating sheet (2), the heating sheet (2) is clamped between the first strap layer (4) and the second strap layer (5), and the magnetic metal terminal (3) is electrically connected to the heating sheet (2).

6. The physiotherapy instrument for tenosynovitis according to claim 1, wherein the wristband assembly comprises a hair side magic tape (7) and a hook side magic tape (8), the hair side magic tape (7) is arranged on the surface of the first strap layer (4) and the hook side magic tape (8) is arranged on the second strap layer (5), so that when the wrist strap wraps around the wrist, the hook side magic tape (8) and the hair side magic tape (7) are adhered.

7. The physiotherapy instrument for tenosynovitis according to claim 1, comprising a heating assembly, wherein the first strap layer (4) and the second strap layer (5) both are cloth layers.

8. The physiotherapy instrument for tenosynovitis according to claim 1, wherein both end parts of the finger part (901) respectively extend at a preset length and are respectively attached to the surface of the wrist strap, one end of each of the two conductive bands (10) is attached to the extended section corresponding to each end part of the finger part (901), and the other end extends into the finger part (901) to fit the inner surface of the finger part (901).

9. The physiotherapy instrument for tenosynovitis according to claim 8, wherein the electrical stimulation assembly comprises a metal rivet, one end of each of the conductive bands (10) and one end of the finger stall strap (9) away from the finger part (901) are riveted to the wrist strap through the metal rivet, and the other end of each of the conductive bands (10) and one end of the finger stall strap (9) close to the finger part (901) are integrally spliced to the surface of the wrist strap.

10. The physiotherapy instrument for tenosynovitis according to claim 1, wherein the controller (1) is provided with a switch button (102), an electrical stimulation gear adjusting button (103), and a heating gear adjusting button (104).
